# EUROPEAN PATENT APPLICATION

(11) **EP 3 991 701 A1**
(43) Date of publication of application: **04.05.2022**
(21) Application number: 20466005.4
(22) Date of filing: 03.12.2020
(51) Int. Cl.: A61F 7/00, A61F 7/08, A61F 7/02

(54) **THERMOTHERAPEUTIC DEVICE**

(30) Priority: 03.11.2020 CZ 20200059
(71) Applicant: Precechtel, Milan, 796 01 Prostejov (CZ)
(72) Inventor: Precechtel, Milan, 796 01 Prostejov (CZ)
(74) Representative: Soukup, Petr

(57) **Abstract**

Thermotherapeutic device consisting of a shell (1) in the shape of a shoe with at least one inner cavity (2) open to the outside with a slip-on opening (21) framed around its circumference by a rim (14). The shell (1) consists of an outer wall (11) and an inner wall (12), between which a thermoactive chamber (13) is formed, wherein both the outer wall (11) and the inner wall (12) are made of flexible, pliable, waterproof and thermally stable material, with the upper rim (14)) of the shell (1) forming a filling neck (3) that contains a filling opening (32), through which the thermoactive chamber (13) is connected to the outside and which is sealable with a cap (34).

## Description

### Field of Technology

The invention falls within the scope of healthcare and applies to a thermotherapeutic device.

### State-of-the-Art

To treat the "cold limb syndrome" and to heat the body, as well as to prevent colds, or as a means to release stress or to relax the body, the most frequently used means currently include electric heaters and blankets, hot water bottles and wellness products, and sometimes containers filled with water. However, electric heaters and blankets need a power supply either from the mains, which may not always be available, or from batteries, which may somewhat complicate handling, including when their power level needs to be checked. Electrical equipment may also present risks due to ageing and degradation of the electrical wiring insulation and the follow-up hazard of a short circuit or electric shock. Furthermore, its use can be problematic in humid or outright wet environments, which also pose a risk of electric shock. Electric blankets are designed to be wrapped around a limb and fixed. As for the use of hot water bottles, this is somewhat inconvenient because of their non-anatomical shape, so they the limb only with one of their surfaces. Finally, wellness products and other water baths require handling heated water, with a risk of spillage and slipping on the wet floor. Water is also in direct contact with the skin of the limb, which may often be undesirable, or it is subsequently necessary to dry the limb. File CN2734187 describes a shoe-shaped electric foot heater which must be connected to the mains and in which only the inner part of the sole is heated. As such, it does not facilitate heating of the limb over its entire surface. File CN87205736 introduces a multipurpose shoe-shaped hot water bottle, which is intended for drying and warming the inside of shoes or socks, and so is unsuitable for actual direct thermotherapy. File CN105496633 presents an electrically heated foot bath for pedicure purposes with a constant water temperature maintained by a thermostat. The device must be connected to the mains and the foot is directly immersed in the water bath. A shoe-shaped electric foot heater with connection to the mains also is described in file CN201624978. A similar device in which an electromagnetic heater is additionally integrated into the water bag heated by the heater, with controllable temperature, is presented in the file CN205548788. File JPH10229996, in turn, introduces a foot heater in the form of a double-walled container with an inner space designed to accommodate the shape of the foot, with the space between the two walls being filled with hot water, and with a sealable refilling cap. This heater is made of solid non-elastic material and is intended solely for installation on the ground, and so can only be used while sitting. File JP2007268230 describes a shoe with a double shell that is designed to be filled with either heating or cooling liquid. The double shell includes reinforcements against deformation and trampling and is also meant for walking. This complicates the double shell design and the reinforcements also add to its weight.

The aim of the present invention is to present a thermotherapeutic device in the form of a sleeve enclosing the feet, with the possibility of both heating and cooling, which uses a liquid medium without the need of connection to a power supply, and is usable with lower limbs in various positions.

### Principle of the Invention

The stated aim is achieved by means of an invention consisting of a thermotherapeutic device in the form of a shoe-shaped shell with at least one inner cavity open to the outside with a slip-on opening framed around its circumference by a rim. The principle of the invention is a shell consisting of an outer wall and an inner wall, with a thermoactive chamber in between, with both the outer wall and the inner wall made of a flexible, pliable, waterproof and thermally stable material. The upper part of the shell's rim is shaped into a filling neck with a filling opening, which connects the thermoactive chamber to the outside and is sealable with a cap.

The convenient design includes the filling opening transitioning into a filling cavity, which connects to the thermoactive chamber.

It is further convenient for the filling opening to be provided with a filling funnel.

The presented invention achieves a novel and better effect in that it is fully functional without the need to be connected to a power supply, is anatomically shaped for the given part of the body, and can be used in different body positions. It can also be used for relaxation purposes or to relax the body, with a positive effect also on the user's state of mind.

### Explanations of Drawings

Concrete examples of the invention design are shown in the attached drawings, where:
- Fig. 1a): is the layout of the basic version of the thermotherapeutic device for one limb;
- Fig. 1b): is a longitudinal section of the device from Fig. 1a) in the A-A plane;
- Fig. 2a): is the layout of an alternative version of the thermotherapeutic device for one limb;
- Fig. 2b): is a longitudinal section of the device from Fig. 2a) in the A-A plane;
- Fig. 3a): is the layout of the basic version of the thermotherapeutic device for two limbs;
- Fig. 3b): is a longitudinal section of the device from Fig. 3a) in the A-A plane;
- Fig. 4a): is the layout of an alternative version of the thermotherapeutic device for two limbs;
- Fig. 4b): is a longitudinal section of the device from Fig. 4a) in the A-A plane;
- Fig. 5a): is the layout of another alternative version of the thermotherapeutic device for two limbs;
- Fig. 5b): is a longitudinal section of the device from Fig. 5a) in the A-A plane;
- Fig. 5c): is a longitudinal section of the device from Fig. 5a) in the B-B plane;
- Fig. 6a): is the layout of another alternative version of the thermotherapeutic device for two limbs;
- Fig. 6b): is a longitudinal section of the device from Fig. 6a) in the A-A plane;
- Fig. 6c): is a longitudinal section of the device from Fig. 6a) in the B-B plane;
- Fig. 7a): is the layout of an alternative version of the shape of the thermotherapeutic device for one limb;
- Fig. 7b): is a longitudinal section of the device from Fig. 7a) in the A-A plane;
- Fig. 8a): is the layout of an alternative version of the shape of the thermotherapeutic device for two limbs;
- Fig. 8b): is a longitudinal section of the device from Fig. 8a) in the A-A plane;
- Fig. 9a): is the layout of an alternative version of the shape of the thermotherapeutic device for one limb;
- Fig.9b): is a longitudinal section of the device from Fig. 9a) in the A-A plane;
- Fig. 9c): is a longitudinal section of the device from Fig. 9a) in the B-B plane.

The drawings illustrating the presented invention and the subsequently described examples of concrete versions do not in any way limit the scope of protection stipulated in the definition, but merely illustrate the principle of the invention.

### Detailed Description of the Invention

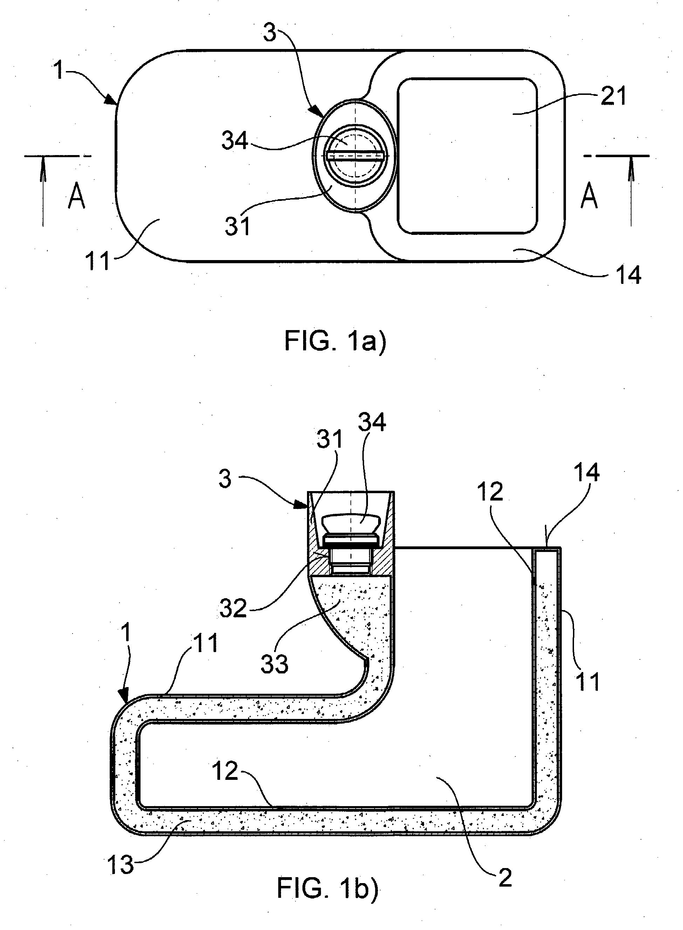

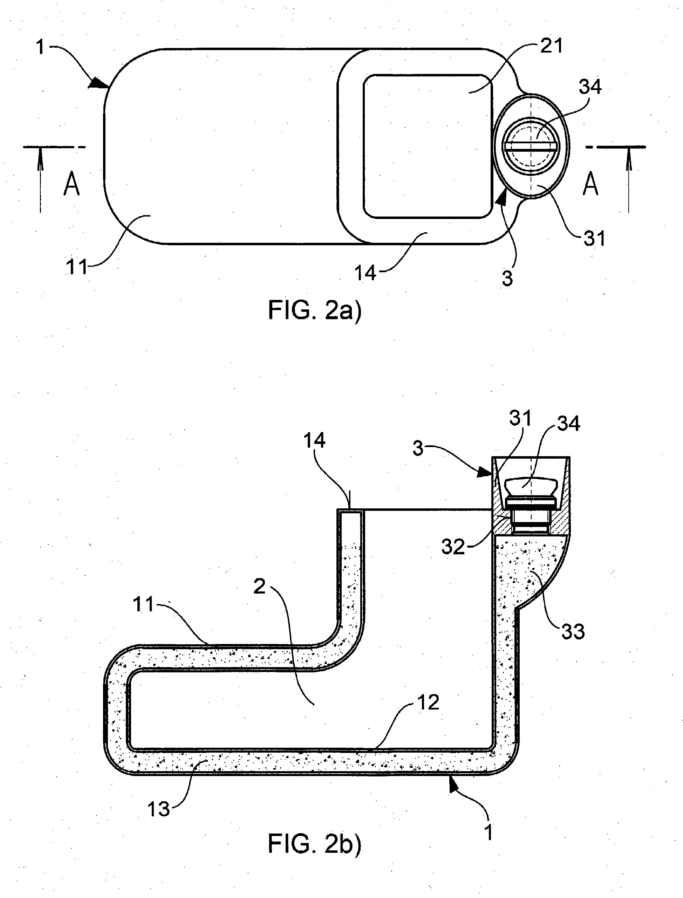

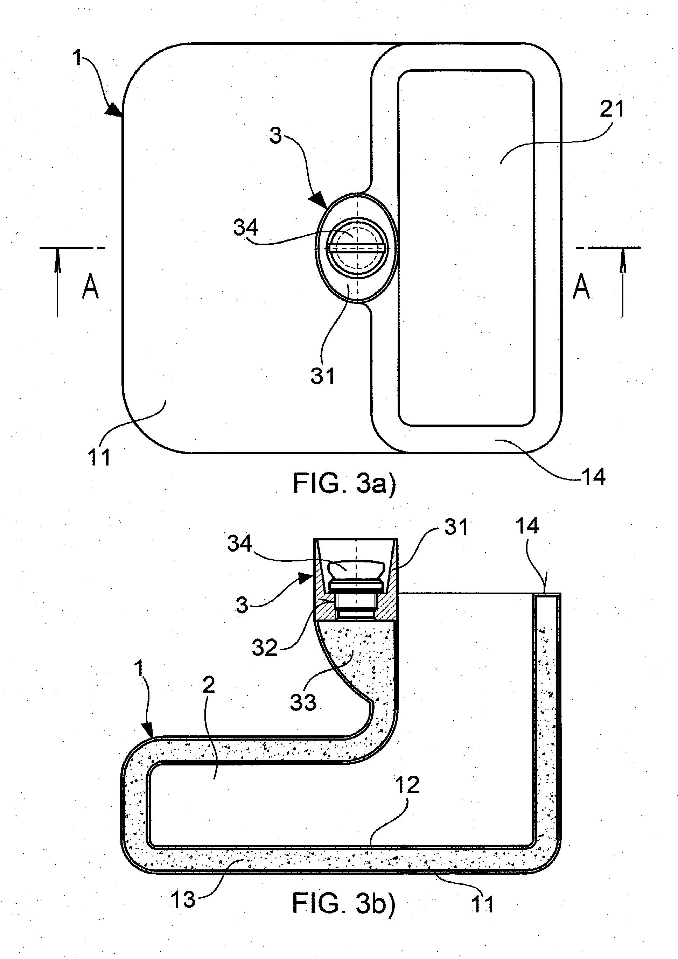

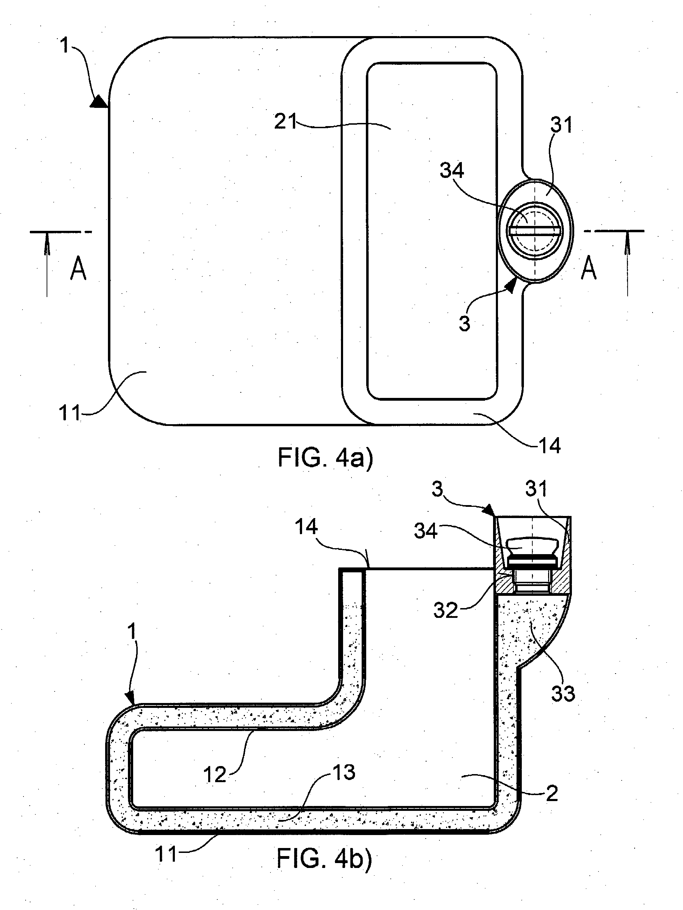

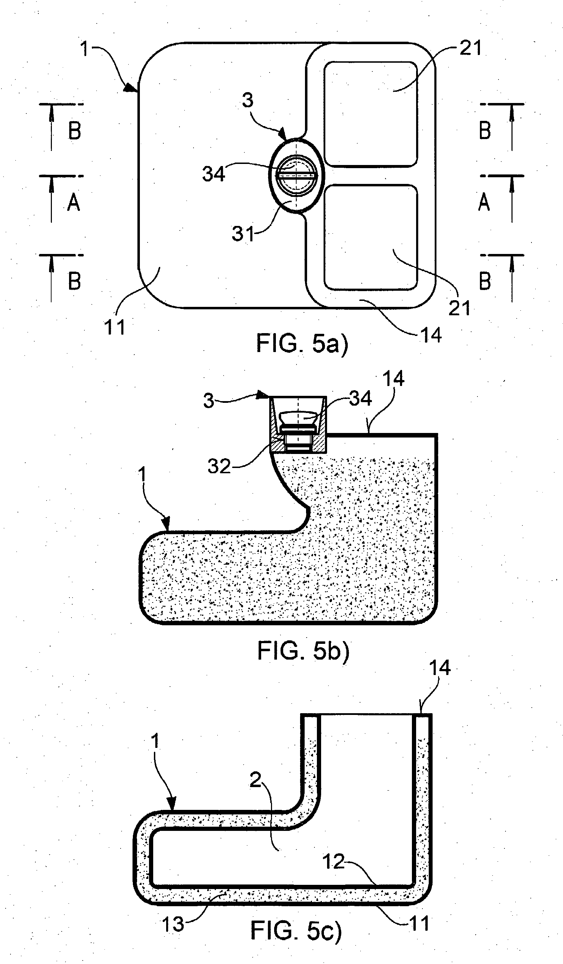

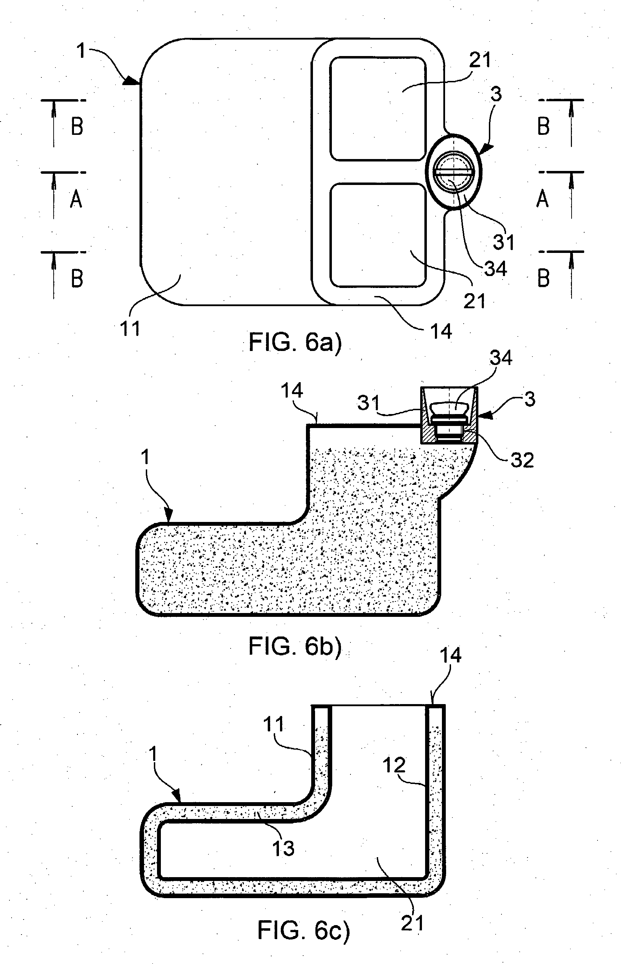

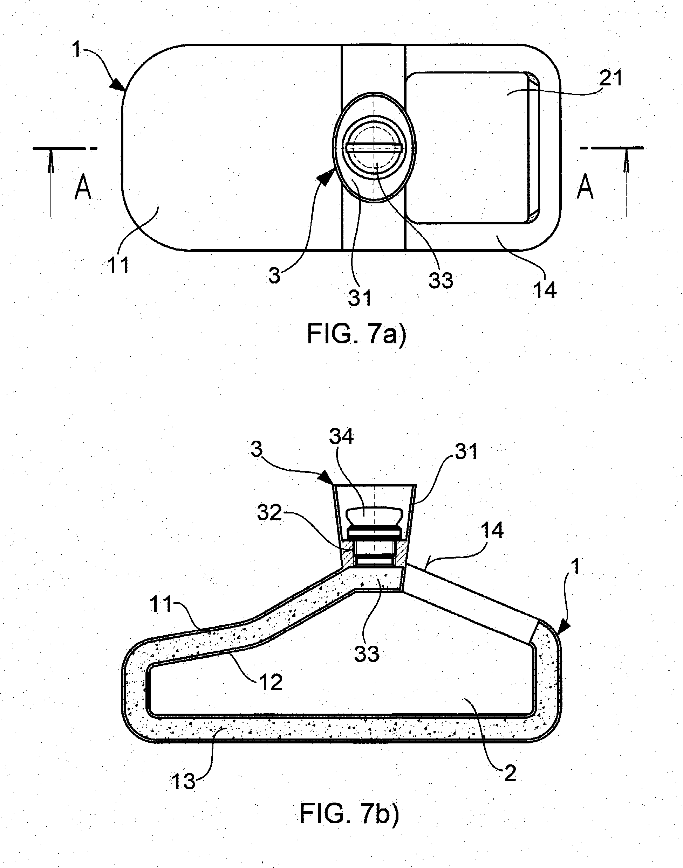

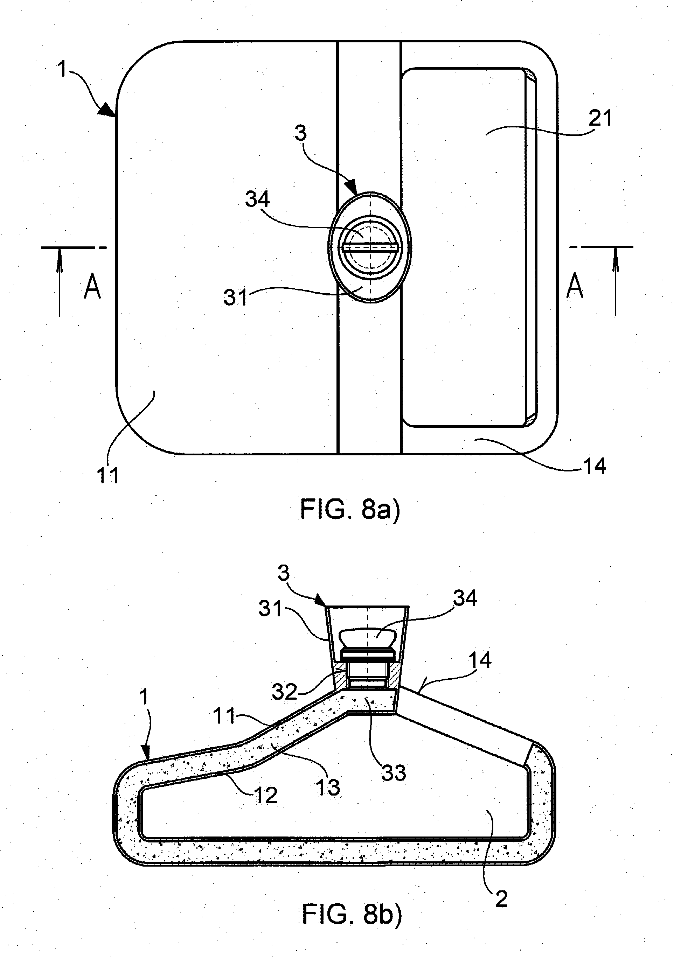

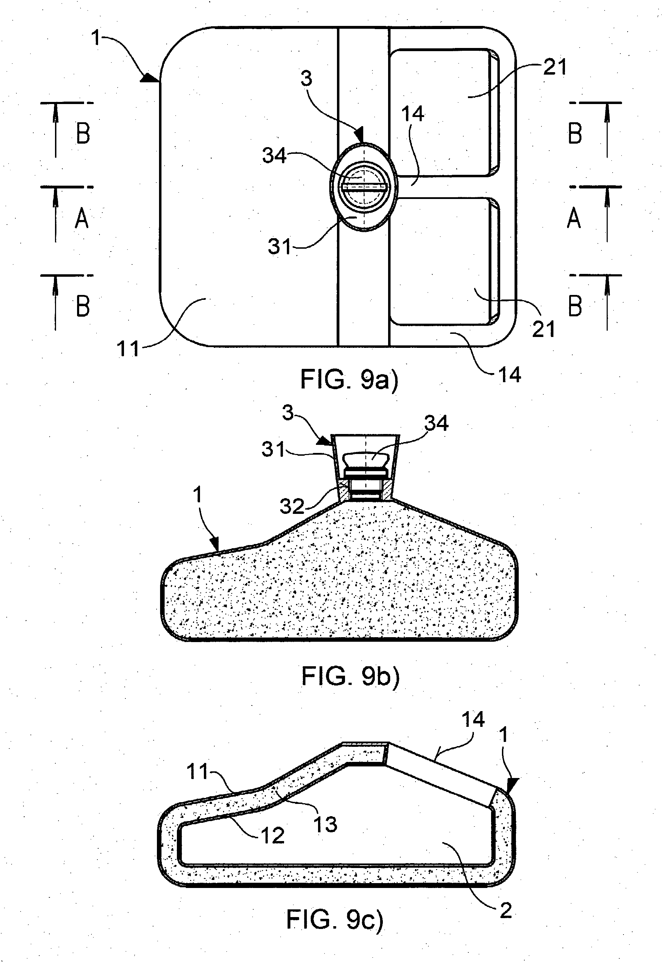

In accordance with Figs. 1 to 4, the thermotherapeutic device consists of a shoe-shaped shell 1 forming the inner cavity 2 designed to accommodate one or two lower limbs. The inner cavity 2 opens upwards to the outside by a vertically arranged slip-on opening 21, which is framed around its circumference by the upper rim 14 of the shell 1. The shell 1 consists of an outer wall 11 and an inner wall 12, between which a thermoactive chamber 13 is formed for liquid medium. Both the outer layer 11 and the inner layer 12 are made of flexible, pliable, waterproof, thermally stable and thermally conductive material. In the upper rim 14 of the shell 1, a filling neck 3 is formed, consisting of the filling opening 32 transitioning into the filling cavity 33 and provided with a filling funnel 31, with the filling cavity 33 connected to the thermoactive chamber 13. The filling opening 32is fitted with a cap34. The cap 34 is threaded or is provided with a bayonet fitting. The filling neck 3 can be located in the front part of the upper rim 14 closer to the instep, as shown in Fig. 1 and Fig. 3, or in its rear part closer to the heel, as shown in Fig. 2 and Fig. 4. The described design is not the only variant possible. As shown in Figs. 5 and 6, the thermotherapeutic device may comprise two inner cavities 2, one for each foot. Figs. 7 to 9 then show alternative shapes of the thermotherapeutic device.

A heating or cooling medium, typically water, is poured into the filling funnel 31 in a volume according to the size of the sleeve, and then flows through the filling opening 32 into the filling cavity 33 and from there on into the thermoactive chamber 13. The amount of the medium to be filled in must be such that the filling cavity 33 is not completely filled and, subsequently, by way of pressure on the outer wall 11. excess air is forced out of the filling cavity 33, and the filling opening is closed with the cap 34. The thermotherapeutic device is then fitted onto the lower limb either in the supine or in the sitting position, or in another body position.

### Industrial Usability

The presented invention can be used in thermotherapy, either when heating is required or when the body needs to be cooled, or for relaxation purposes.

## Claims

1. Thermotherapeutic device consisting of a shell (1) in the shape of a shoe with at least one inner cavity (2) open to the outside with a slip-on opening (21) framed around its circumference by a rim (14), **wherein** the shell (1) consists of an outer wall (11) and an inner wall (12), between which a thermoactive chamber (13) is formed, wherein both the outer wall (11) and the inner wall (12) are made of flexible, pliable, waterproof and thermally stable material, with the upper rim (14)) of the shell (1) forming a filling neck (3) that contains a filling opening (32), through which the thermoactive chamber (13) is connected to the outside and which is sealable with a cap (34).

2. Thermotherapeutic device according to claim 1, **wherein** the filling opening (32) opens into a filling cavity (33) which is connected to the thermoactive chamber (13).

3. Thermotherapeutic device according to claim 1 or 2, **wherein** the filling opening (32) is provided with a filling funnel (31).
